(19) Europäisches Patentamt
European Patent Office
Office européen des brevets

(11) Veröffentlichungsnummer: **0 483 627 A1**

(12) **EUROPÄISCHE PATENTANMELDUNG**

(21) Anmeldenummer: **91117882.0**

(22) Anmeldetag: **19.10.91**

(51) Int. Cl.5: **C07C 309/42**, C07C 309/46, C07C 309/14, C07C 215/24, C25D 3/12

(30) Priorität: **02.11.90 DE 4034788**

(43) Veröffentlichungstag der Anmeldung: **06.05.92 Patentblatt 92/19**

(84) Benannte Vertragsstaaten: **DE FR GB IT**

(71) Anmelder: **BASF Aktiengesellschaft**
**Carl-Bosch-Strasse 38**
**W-6700 Ludwigshafen(DE)**

(72) Erfinder: **Greif, Norbert, Dr.**
**3 Im Woogtal**
**W-6719 Bobenheim(DE)**
Erfinder: **Oppenlaender, Knut, Dr.**
**Otto-Dill-Strasse 23**
**W-6700 Ludwigshafen(DE)**
Erfinder: **Fischer, Martin, Dr.**
**Elbinger Weg 1**
**W-6700 Ludwigshafen(DE)**
Erfinder: **Tschang Chung-Ji, Dr.**
**Hinterbergstrasse 31**
**W-6702 Bad Duerkheim(DE)**
Erfinder: **Glaser, Klaus**
**T3, 8**
**W-6800 Mannheim(DE)**

(54) **Verfahren zur Herstellung vernickelter Formteile.**

(57) Herstellung vernickelter Formteile durch galvanisches Abscheiden von Nickel aus wäßrig-sauren Bädern, die als wesentliche Bestandteile ein oder mehrere Nickelsalze, eine oder mehrere anorganische Säuren und ein oder mehrere Glanzmittel enthalten, indem man als Glanzmittel 2-Hydroxy-3-buten-Reste enthaltende Verbindungen der allgemeinen Formel I

$$(CH_2{=}CH{-}\underset{\displaystyle OH}{\underset{|}{C}H}{-}CH_2{-})_n\,A \qquad \text{I}$$

in der n für 1, 2 oder 3 steht und die Variable A folgende Bedeutungen hat
für n = 1
- eine Hydroxylgruppe
- eine Gruppe der Formel $-OR^1$, wobei $R^1$ für $C_1$- bis $C_4$-Alkyl, welches zusätzlich eine Sulfogruppe tragen kann, Phenyl, 3- oder 4-Sulfophenyl, α- oder β-Naphthyl, 3-, 4-, 5-, 6- oder 7-Sulfo-α-naphthyl oder 4-, 5-, 6-, 7- oder 8-Sulfo-β-naphthyl steht
- eine phosphorhaltige Gruppe der Formel $-O-PO(OX)H$ oder $-O-PO(OX)_2$, wobei X Wasserstoff oder ein Alkalimetall- oder Ammoniumkation bedeutet
- eine Sulfogruppe der Formel $-SO_3X$
- eine Aminogruppe der Formel $-NH_2$, $-NHR^1$ oder $-N(R^1)_2$, wobei beim Vorliegen von zwei Resten $R^1$ am N-Atom diese gleich oder verschieden sein können

für n = 2
- eine Aminogruppe der Formel -NH- oder $-NR^1-$
- ein divalentes O-Atom

für n = 3

EP 0 483 627 A1

- ein trivalentes N-Atom

einsetzt.

Die vorliegende Erfindung betrifft ein verbessertes Verfahren zur Herstellung vernickelter Formteile durch galvanisches Abscheiden von Nickel aus wäßrig-sauren Bädern, die als wesentliche Bestandteile ein oder mehrere Nickelsalze, eine oder mehrere anorganische Säuren und ein oder mehrere Glanzmittel enthalten, wobei diese Glanzmittel teilweise neue Verbindungen darstellen. Weiterhin betrifft die Erfindung eine hierzu verwendbare Glanzmittelmischung.

Es ist bekannt, daß saure Nickelelektrolyte in geringer Menge organische Substanzen enthalten müssen, wenn bei der galvanischen Nickelabscheidung eine glänzende, duktile und an der Oberfläche ebene Abscheidung des Metalls erzielt werden soll. Derartige Glanzmittel, die man in der Regel in primäre Glanzmittel ("Glanzträger") und sekundäre Glanzmittel ("Glanzbildner") unterteilt, werden üblicherweise als Kombination aus mehreren dieser Mittel eingesetzt, um die Wirkung zu steigern. Trotzdem ist der so erreichte Glanz immer noch verbesserungsbedürftig.

In der DE-B 11 91 652 (1) werden ein- oder mehrkernige heterocyclische Stickstoffbasen vom aromatischen Typ in quaternisierter Form wie Pyridiniumsalze, z.B. 2-Pyridinium-1-sulfatoethan, als Einebnungsmittel, d.h. Glanzmittel, für saure galvanische Nickelbäder beschrieben. Diese Mittel werden zusammen mit üblichen Grundglanzmitteln wie Benzol-m-disulfonsäure, Diaryldisulfimiden oder Sulfonamiden verwendet.

In der Literaturstelle "Praktische Galvanotechnik", Eugen G. Lenze Verlag, Saulgau 4. Auflage 1984, Seite 268 bis 271 (2) werden Substanzklassen für übliche Glanzmittel für Nickelelektrolyte beschrieben. Eine Einteilung in primäre und sekundäre Glanzmittel und Einebner wird zwar vorgenommen, es wird aber gleichzeitig darauf hingewiesen, daß diese Einteilung nicht immer eindeutig vorgenommen werden kann. Als glanzerzeugende Substanzklassen werden genannt:

- Sulfonimide, z.B. Benzoesäuresulfimid
- Sulfonamide
- Benzolsulfonsäuren, z.B. Mono-, Di- und Tribenzolsulfonsäure
- Naphthalinsulfonsäuren, z.B. Mono-, Di- und Trinaphthalinsulfonsäure
- Alkylsulfonsäuren
- Sulfinsäure
- Arylsulfonsulfonate
- aliphatische Verbindungen mit Ethylen- und/oder Acetylenbindungen, z.B. Butindiol
- ein- und mehrkernige stickstoffhaltige Heterocyclen, welche noch weitere Heteroatome wie Schwefel oder Selen enthalten können
- Cumarin
- Amine und quaternäre Ammoniumverbindungen als Einebnungsmittel
- Saccharin.

In der Praxis werden üblicherweise als Glanzbildner Alkenylsulfonsäuren wie Natriumvinylsulfonat oder Natriumallylsulfonat in Kombination mit anderen Glanzbildnern wie Propargylalkohol, 2-Butin-1,4-diol, Propinsulfonsäure oder 2-Pyridinium-1-sulfatoethan verwendet.

Der Erfindung lag daher ein verbessertes Verfahren zur Herstellung vernickelter Formteile mit stärkerem Glanz bei gleich guter Duktilität und Ebenheit an der Oberfläche des Formteils als Aufgabe zugrunde.

Demgemäß wurde ein Verfahren zur Herstellung vernickelter Formteile durch galvanisches Abscheiden von Nickel aus wäßrig-sauren Bädern, die als wesentliche Bestandteile ein oder mehrere Nickelsalze, eine oder mehrere anorganische Säuren und ein oder mehrere Glanzmittel enthalten, gefunden, welches dadurch gekennzeichnet ist, daß man als Glanzmittel 2-Hydroxy-3-buten-Reste enthaltende Verbindungen der allgemeinen Formel I

$$(CH_2{=}CH{-}\underset{\displaystyle OH}{\underset{|}{C}}H{-}CH_2{-})_n\,A \qquad \text{I}$$

in der n für 1, 2 oder 3 steht und die Variable A folgende Bedeutungen hat

für n = 1

- eine Hydroxylgruppe
- eine Gruppe der Formel $-OR^1$, wobei $R^1$ für $C_1$- bis $C_4$-Alkyl, welches zusätzlich eine Sulfogruppe tragen kann, Phenyl, 3- oder 4-Sulfophenyl, $\alpha$- oder $\beta$-Naphthyl, 3-, 4-, 5-, 6- oder 7-Sulfo-$\alpha$-naphthyl oder 4-, 5-, 6-, 7- oder 8-Sulfo-$\beta$-naphthyl steht
- eine phosphorhaltige Gruppe der Formel $-O-PO(OX)H$ oder $-O-PO(OX)_2$, wobei X Wasserstoff oder ein Alkalimetall- oder Ammoniumkation bedeutet

- eine Sulfogruppe der Formel $-SO_3X$
- eine Aminogruppe der Formel $-NH_2$, $-NHR^1$ oder $-N(R^1)_2$, wobei beim Vorliegen von zwei Resten $R^1$ am N-Atom diese gleich oder verschieden sein können

für n = 2

- eine Aminogruppe der Formel -NH- oder $-NR^1-$
- ein divalentes O-Atom

für n = 3

- ein trivalentes N-Atom

einsetzt.

Steht der Rest $R^1$ für $C_1$- bis $C_4$-Alkyl, welches zusätzlich eine Sulfogruppe tragen kann, sind hierunter vor allem die Reste Methyl, Ethyl, n-Propyl, iso-Propyl, n-Butyl, iso-Butyl, sek.-Butyl, tert.-Butyl, Sulfomethyl, 2-Sulfoethyl, 3-Sulfopropyl und 4-Sulfobutyl zu verstehen, wobei die Sulfogruppe üblicherweise als freie Säure, als Alkalimetallsalz, insbesondere Natrium- oder Kaliumsalz, oder als Ammoniumsalz vorliegt. Besonderes gute Resultate erzielt man hiervon mit der 2-Sulfoethylgruppe.

Bei Verbindung I mit einer phosphorhaltigen Gruppe der Formel -O-PO(OX)H handelt es sich um Phosphorigsäuremonoester bzw. Salze hiervon und bei Verbindungen I mit einer phosphorhaltigen Gruppe der Formel $-O-PO(OX)_2$ um Phosphorsäuremonoester bzw. Salze hiervon.

Die Variable X bedeutet Wasserstoff oder ein Alkalimetallkation, insbesondere Natrium oder Kalium, oder das Ammoniumkation. Da die Verbindungen I erfindungsgemäß in saurem Medium, insbesondere schwach saurem Medium, eingesetzt werden, liegt naturgemäß ein Gleichgewicht zwischen den Alkalimetall- bzw. Ammoniumsalzen und den zugehörigen freien Säuren vor.

In einer bevorzugten Ausführungsform der Erfindung setzt man Verbindungen I ein, bei denen n für 1 oder 3 steht und die Variable A folgende Bedeutungen hat

für n = 1

- eine Hydroxylgruppe
- eine Gruppe der Formel $-OR^2$, wobei $R^2$ für 2-Sulfoethyl, 3-Sulfophenyl und 4-Sulfophenyl steht
- eine Sulfogruppe der Formel $-SO_3X$
- eine Aminogruppe der Formel $-NHR^2$

für n = 3

- ein trivalentes N-Atom.

Weiterhin sind Gegenstand der vorliegenden Erfindung neue 2-Hydroxy-3-buten-Reste enthaltende Verbindungen der allgemeinen Formel Ia

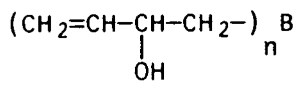

Ia

in der n für 1, 2 oder 3 steht und die Variable B folgende Bedeutungen hat:

für n = 1

- eine Gruppe der Formel $-OR^3$, wobei $R^3$ für $C_1$- bis $C_4$-Alkyl, welches zusätzlich eine Sulfogruppe trägt, 3- oder 4-Sulfophenyl, $\alpha$- oder $\beta$-Naphthyl, 3-, 4-, 5-, 6- oder 7-Sulfo-$\alpha$-naphthyl oder 4-, 5-, 6-, 7- oder 8-Sulfo-$\beta$-naphthyl steht
- eine phosphorhaltige Gruppe der Formel -O-PO(OX)H oder $-O-PO(OX)_2$, wobei X Wasserstoff oder ein Alkalimetall- oder Ammoniumkation bedeutet
- eine Aminogruppe der Formel $-NHR^3$
- eine Aminogruppe der Formel $-N(R^4)_2$ mit zwei gleichen Resten $R^4$, wobei $R^4$ für $R^3$ oder für Phenyl steht

für n = 2

- eine Aminogruppe der Formel -NH-
- eine Aminogruppe der Formel $-NR^3-$

für n = 3

- ein trivalentes N-Atom

steht der Reste $R^3$ für $C_1$- bis $C_4$-Alkyl, welches zusätzlich eine Sulfogruppe trägt, sind hierunter vor allem die Reste Sulfomethyl, 2-Sulfoethyl, 3-Sulfopropyl und 4-Sulfobutyl zu verstehen, wobei die Sulfogruppe üblicherweise als freie Säure, als Alkalimetallsalz, insbesondere Natrium- oder Kaliumsalz, oder als Ammoniumsalz vorliegt.

Die Verbindungen 1 bzw. Ia stellt man zweckmäßigerweise durch Umsetzung von vinyloxiran, 4-Chlor-3-hydroxy-1-buten oder 4-Brom-3-hydroxy-1-buten mit den entsprechende nucleophile Gruppen aufweisenden Reaktionspartnern im entsprechenden molaren Verhältnis her. Derartige Reaktionspartner sind beispielsweise:

- Wasser
- $C_1$- bis $C_4$-Alkanole
- Salze der Hydroxymethansulfonsäure, 2-Hydroxyethansulfonsäure (Isethionsäure), 3-Hydroxypropansulfonsäure oder 4-Hydroxybutansulfonsäure
- Phenol sowie $\alpha$- oder $\beta$-Naphthol
- Salze von 3- oder 4-Sulfophenol sowie von 3-, 4-, 5-, 6- oder 7-Sulfo-$\alpha$-naphthol oder 4-, 5-, 6-, 7- oder 8-Sulfo-$\beta$-naphthol
- phosphorige Säure oder Hydrogenphosphite
- Phosphorsäure oder Hydrogen- oder Dihydrogenphosphate
- schwefelige Säure oder Hydrogensulfite
- Ammoniak
- Mono- oder Dialkylamine mit 1 bis 4 C-Atomen pro Alkylgruppe
- Salze der Aminomethansulfonsäure, 2-Aminoethansulfonsäure (Taurin), 3-Aminopropansulfonsäure oder 4-Aminobutansulfonsäure
- Anilin sowie $\alpha$- oder $\beta$-Naphthylamin
- Metanilsäure oder Sulfanilsäure und 3-, 4-, 5-, 6- oder 7-Sulfo-$\alpha$-naphthylamin oder 4-, 5-, 6-, 7- oder 8-Sulfo-$\beta$-naphthylamin sowie Salze dieser Verbindungen.

Die Umsetzung wird in der Regel unter den für Epoxide und Halogenhydrine üblichen Bedingungen durchgeführt, wie sie beispielsweise in den Literaturstellen M.S. Malinovskii, "Epoxides and their Derivatives", Israel Program for Scientific Translations, Jerusalem, 1965, oder Houben-Weyl, "Methoden der Organischen Chemie", Bd. VI/3 (Sauerstoffverbindungen I, Teil 3), Thieme Verlag Stuttgart, 1965, beschrieben sind.

In einer bevorzugten Ausführungsform der Erfindung wird neben den Verbindungen I mindestens ein weiteres Glanzmittel verwendet. Da den Verbindungen I überwiegend der Wirkungscharakter von primären Glanzmitteln zukommt, ergeben sie in Kombination mit sekundären Glanzmitteln wie Propargylalkohol, 2-Butin-1,4-diol, Propinsulfonsäure oder vorzugsweise einer ein- oder mehrkernigen heterocyclischen Stickstoffbase vom aromatischen Typ in quaternisierter Form, z.B. 2-Pyridinium-1-sulfatoethan, einen besonders hohen Glanz. Daneben ist aber auch eine Wirkungsverstärkung mit bestimmten primären Glanzmitteln wie Saccharin festzustellen.

Die verwendeten wäßrig-sauren Nickelelektrolyt-Bäder enthalten ein oder meist mehrere Nickelsalze, z.B. Nickelsulfat und Nickelchlorid, eine oder mehrere anorganische Säuren, vorzugsweise Borsäure und Schwefelsäure, als Glanzmittel die Verbindungen I allein oder vorzugsweise in Kombination mit weiteren üblichen Glanzmitteln sowie gegebenenfalls weitere übliche Hilfsmittel und Zusätze in den hierfür üblichen Konzentrationen, z.B. Netzmittel und Porenverhütungsmittel. Gebräuchliche wäßrig-saure Nickelelektrolyte ("Watts-Elektrolyte") haben die folgende Grundzusammensetzung:

200 bis 350 g/l $NiSO_4 \cdot 7\ H_2O$
30 bis 150 g/l $NiCl_2 \cdot 6\ H_2O$
30 bis 50 g/l $H_3BO_3$.

Der pH-Wert der Elektrolyt-Bäder liegt üblicherweise zwischen 3 und 6, vorzugsweise zwischen 4 und 5. Zur Einstellung dieses pH-Wertes dient zweckmäßigerweise eine starke Mineralsäure, vorzugsweise Schwefelsäure.

Die Verbindungen I liegen in den Elektrolyt-Bädern in der Regel in Konzentrationen zwischen 0,1 und 2,0 g/l, vorzugsweise zwischen 0,2 und 1,3 g/l, vor. Die Konzentrationen weiterer üblicher Glanzmittel liegen normalerweise ebenfalls im Bereich von jeweils 0,1 bis 2,0 g/l.

Mit den beschriebenen Nickelelektrolyt-Bädern können vor allem Nickelüberzüge auf Formteilen aus Stahl, daneben aber auch auf Formteilen aus anderen Materialien, beispielsweise Messing, die wie üblich vorbehandelt worden sind, galvanisch erzeugt werden. Hierzu arbeitet man in der Regel bei Temperaturen von 30 bis 80 °C, vorzugsweise 40 bis 60 °C.

Gegenstand der vorliegenden Erfindung ist weiterhin eine Glanzmittelmischung für wäßrig-saure galvanische Bäder zur Abscheidung von Nickel, welche als wesentliche Bestandteile die Verbindungen I und eine ein- oder mehrkernige heterocyclische Stickstofffbase vom aromatischen Typ in quaternisierter Form in den oben genannten Konzentrationen enthält.

Die erfindungsgemäß verwendeten Verbindungen I zeichnen sich durch eine außerordentlich hohe Glanzbildung aus. Man erreicht mit ihnen einen stärkeren Glanz als mit den üblichen Glanzmitteln und dies teilweise sogar bei niedrigerer Dosierung in den Nickelelektrolyt-Bädern.

Herstellungsbeispiele Nr. 1 bis 6

Beispiel 1

Herstellung von 1,2-Dihydroxy-3-buten

32,7 g Vinyloxiran, 70 g Wasser und 0,5 g 96 gew.-%ige Schwefelsäure als Katalysator wurden 5 Stunden bei 85 bis 90 °C gerührt. Danach wurde die Schwefelsäure mit 50 gew.-%iger Natronlauge neutralisiert. Geringe Mengen an nicht umgesetztem Vinyloxiran wurden zusammen mit überschüssigem Wasser im Wasserstrahlvakuum bei 50 °C abdestilliert. Man erhielt 40 g Produkt in Form einer viskosen Flüssigkeit.

Beispiel 2

Herstellung von Natrium-4-(2'-hydroxy-3'-buten-1'-yloxy)-benzolsulfonat

267,7 g 65 gew.-%iger p-Phenolsulfonsäure wurden mit 100 g Wasser verdünnt und mit 80 g 50 gew.-%iger Natronlauge versetzt. Danach wurden bei 70 bis 80 °C unter Rühren 70 g Vinyloxiran im Laufe von 30 Minuten zugetropft. Es wurde 8 Stunden bei derselben Temperatur weitergerührt. Im Wasserstrahlvakuum wurde dann das Wasser entfernt, wobei 195 g Produkt zurückblieben. Das Produkt wurde wieder mit Wasser aufgenommen und auf eine Konzentration von 50 Gew.-% eingestellt.

Beispiel 3

Herstellung von Natrium-2-hydroxy-3-butensulfonat

Zu einer Mischung aus 80,4 g 39 gew.-%iger wäßriger Natriumhydrogensulfit-Lösung und 23,6 g Wasser wurden bei 80 °C unter Rühren 21,5 g Vinyloxiran im Laufe von 20 Minuten getropft. Anschließend wurde bei derselben Temperatur 8 Stunden weitergerührt. Geringe Mengen an nicht umgesetztem Vinyloxiran sowie das Wasser wurden im Wasserstrahlvakuum entfernt. Es blieben 40 g Produkt zurück. Dieses wurden mit Wasser aufgenommen und auf eine Konzentration von 50 Gew.-% eingestellt.

Beispiel 4

Herstellung von Tris(2-hydroxy-3-buten-1-yl)amin

Zu einer Mischung aus 40,8 g 25 gew.-%igem Ammoniakwasser und 170 g Wasser wurden bei 70 bis 80 °C unter Rühren 126 g Vinyloxiran im Laufe von 20 Minuten getropft. Anschließend wurde bei derselben Temperatur 5 Stunden weitergerührt. Danach wurde das Reaktionsgemisch im Wasserstrahlvakuum auf 172 g eingeengt. Man erhielt eine viskose Flüssigkeit mit einem Produktgehalt von 78 Gew.-%.

Beispiel 5

Herstellung von Natrium-2-N-(2'-hydroxy-3'-buten-1'-yl)amino-ethansulfonat

Zu 313 g einer 47 gew.-%igen wäßrigen Lösung des Natriumsalzes von 2-Aminoethansulfonsäure (Taurin) wurden bei 70 bis 80 °C unter Rühren 70 g Vinyloxiran im Laufe von 30 Minuten getropft. Anschließend wurde bei derselben Temperatur 6 Stunden weitergerührt. Danach wurde im Wasserstrahlvakuum das Wasser entfernt. Dabei ergaben sich 218 g Produkt. Das Produkt wurde erneut mit Wasser aufgenommen, wobei ein Produktgehalt von 50 Gew.-% eingestellt wurde.

Beispiel 6

Herstellung von Natrium-2-N,N-bis(2'-hydroxy-3'-butenl-1'-yl)aminoethansulfonat

Das Produkt wurde analog zu Beispiel 5 mit der doppelten Menge Vinyloxiran hergestellt. Man erhielt nach erfolgter Umsetzung und Einengung des Reaktionsgemisches im Wasserstrahlvakuum 287 g Produkt, das erneut mit Wasser aufgenommen und auf einen Produktgehalt von 50 Gew.-% eingestellt wurde.

Beispiel 7

Herstellung von Natrium-4-N-(2'-hydroxy-3'-buten-1'-yl)amino-benzol-sulfonat

Zu einer Mischung aus 173 g 4-Aminobenzolsulfonsäure (Sulfanilsäure), 200 g Wasser und 80 g 50 gew.-%iger wäßriger Natronlauge wurden bei 70 bis 80 °C unter Rühren 70 g Vinyloxiran im Laufe von 30 Minuten getropft. Anschließend wurde bei derselben Temperatur 4 Stunden weitergerührt. Man erhielt 520 g einer Lösung mit einem Produktgehalt von 53 Gew.-%.

Anwendungsbeispiele Nr. 8 bis 14 und Vergleichsbeispiele A und B

Die in den Beispielen 1 bis 7 hergestellten Produkte wurden als Glanzmittel in schwach sauren galvanischen Bädern zur Abscheidung von Nickel eingesetzt.

Der verwendete wäßrige Nickelelektrolyt hatte folgende Zusammensetzung:

300 g/l $NiSO_4 \cdot 7 H_2O$
60 g/l $NiCl_2 \cdot 6 H_2O$
45 g/l $H_3BO_3$
2 g/l Saccharin
0,3 g/l 2-Pyridinium-1-sulfatoethan
X g/l Glanzmittel gemäß Tabelle
0,5 g/l eines Fettalkoholderivates der Formel $C_{12}H_{25}/C_{14}H_{29}$-O-$(CH_2CH_2O)_2$-$SO_3Na$ als Netzmittel

Der pH-Wert des Elektrolyten wurde mit Schwefelsäure auf 4,2 eingestellt.

Es wurden Messingbleche verwendet, die vor der Beschichtung mit Nickel in einem alkalischen Elektrolyten nach den üblichen Methoden kathodisch entfettet wurden. Die Nickelabscheidung erfolgte in einer 250 ml-Hull-Zelle bei 55 °C und einer Stromstärke von 2A während eines Zeitraumes von 10 Minuten. Anschließend wurden die Bleche mit Wasser gespült und mit Preßluft getrocknet.

Die folgende Tabelle zeigt die Ergebnisse dieser Versuche. Man erkennt, daß mit den erfindungsgemäß verwendeten Glanzmitteln bzw. Glanzmittelmischungen ein stärkerer Glanz erreicht wird als mit den Mitteln des Standes der Technik und dies teilweise sogar bei niedrigerer Dosierung in den Nickelelektrolyt-Bädern.

Tabelle

Prüfergebnisse der galvanischen Nickelabscheidung

| Beispiel Nr. | Glanzmittel aus Beispiel Nr. | Konzentration x [g/l] | Glanz [Note] |
|---|---|---|---|
| 8 | 1 | 0,3 | 4-5 |
| 9 | 2 | 0,8 | 4-5 |
| 10 | 3 | 0,8 | 4-5 |
| 11 | 4 | 0,3 | 5 |
| 12 | 5 | 0,6 | 5 |
| 13 | 6 | 0,8 | 5 |
| 14 | 7 | 1,2 | 4-5 |
| zum Vergleich: | | | |
| A | Natriumvinylsulfonat | 0,8 | 4 |
| B | Natriumallylsulfonat | 0,7 | 4 |

Benotungsschema für den Glanz:
5 = hervorragend (perfekter Spiegelglanz)
4 = gut (nahezu Spiegelglanz)
3 = mäßig
2 = schlecht
1 = kein Glanz

Die Duktilität war bei allen Beispielen Nr. 8 bis 14 wie bei den Vergleichsprüfungen A und B sehr gut.

Außer bei Beispiel 8, bei dem eine Spur einer überinhibierung festzustellen war, konnten keinerlei Störungen bei der Nickelabscheidung beobachtet werden.

## Patentansprüche

**1.** Verfahren zur Herstellung vernickelter Formteile durch galvanisches Abscheiden von Nickel aus wäßrig-sauren Bädern, die als wesentliche Bestandteile ein oder mehrere Nickelsalze, eine oder mehrere anorganische Säuren und ein oder mehrere Glanzmittel enthalten, dadurch gekennzeichnet, daß man als Glanzmittel 2-Hydroxy-3-buten-Reste enthaltende Verbindungen der allgemeinen Formel I

$$\left(CH_2{=}CH{-}\underset{\displaystyle OH}{\underset{|}{C}}H{-}CH_2{-}\right)_n A \qquad I$$

in der n für 1, 2 oder 3 steht und die Variable A folgende Bedeutungen hat
für n = 1

- eine Hydroxylgruppe
- eine Gruppe der Formel $-OR^1$, wobei $R^1$ für $C_1$- bis $C_4$-Alkyl, welches zusätzlich eine Sulfogruppe tragen kann, Phenyl, 3- oder 4-Sulfophenyl, $\alpha$- oder $\beta$-Naphthyl, 3-, 4-, 5-, 6- oder 7-Sulfo-$\alpha$-naphthyl oder 4-, 5-, 6-, 7- oder 8-Sulfo-$\beta$-naphthyl steht
- eine phosphorhaltige Gruppe der Formel $-O-PO(OX)H$ oder $-O-PO(OX)_2$, wobei X Wasserstoff oder ein Alkalimetall- oder Ammoniumkation bedeutet
- eine Sulfogruppe der Formel $-SO_3X$

- eine Aminogruppe der Formel $-NH_2$, $-NHR^1$ oder $-N(R^1)_2$, wobei beim Vorliegen von zwei Resten $R^1$ am N-Atom diese gleich oder verschieden sein können

für n = 2

- eine Aminogruppe der Formel -NH- oder $-NR^1-$
- ein divalentes O-Atom

für n = 3

- ein trivalentes N-Atom

einsetzt.

**2.** Verfahren zur Herstellung vernickelter Formteile nach Anspruch 1, dadurch gekennzeichnet, daß man Verbindungen I einsetzt, bei denen n für 1 oder 3 steht und die Variable A folgende Bedeutungen hat

für n = 1

- eine Hydroxylgruppe
- eine Gruppe der Formel $-OR^2$, wobei $R^2$ für 2-Sulfoethyl, 3-Sulfophenyl und 4-Sulfophenyl steht
- eine Sulfogruppe der Formel $-SO_3X$
- eine Aminogruppe der Formel $-NHR^2$

für n = 3

- ein trivalentes N-Atom.

**3.** Verfahren zur Herstellung vernickelter Formteile nach Anspruch 1 oder 2, dadurch gekennzeichnet, daß man neben den Verbindungen I mindestens ein weiteres Glanzmittel verwendet.

**4.** Verfahren zur Herstellung vernickelter Formteile nach Anspruch 3, dadurch gekennzeichnet, daß man neben den Verbindungen I eine ein- oder mehrkernige heterocyclische Stickstoffbase vom aromatischen Typ in quaternisierter Form als weiteres Glanzmittel verwendet.

**5.** 2-Hydroxy-3-buten-Reste enthaltende Verbindungen der allgemeinen Formel Ia

$$(CH_2{=}CH{-}\underset{\substack{|\\ OH}}{C}H{-}CH_2{-})_n\,B \qquad \text{Ia}$$

in der n für 1, 2 oder 3 steht und die Variable B folgende Bedeutungen hat

für n = 1

- eine Gruppe der Formel $-OR^3$, wobei $R^3$ für $C_1$- bis $C_4$-Alkyl, welches zusätzlich eine Sulfogruppe trägt, 3- oder 4-Sulfophenyl, $\alpha$- oder $\beta$-Naphthyl, 3-, 4-, 5-, 6- oder 7-Sulfo-$\alpha$-naphthyl oder 4-, 5-, 6-, 7- oder 8-Sulfo-$\beta$-naphthyl steht
- eine phosphorhaltige Gruppe der Formel -O-PO(OX)H oder $-O-PO(OX)_2$, wobei X Wasserstoff oder ein Alkalimetall- oder Ammoniumkation bedeutet
- eine Aminogruppe der Formel $-NHR^3$
- eine Aminogruppe der Formel $-N(R^4)_2$ mit zwei gleichen Resten $R^4$, wobei $R^4$ für $R^3$ oder für Phenyl steht

für n = 2

- eine Aminogruppe der Formel -NH-
- eine Aminogruppe der Formel $-NR^3-$

für n = 3

- ein trivalentes N-Atom.

**6.** Glanzmittelmischung für wäßrig-saure galvanische Bäder zur Abscheidung von Nickel, enthaltend als wesentliche Bestandteile Verbindungen I gemäß Anspruch 1 oder 2 und eine ein- oder mehrkernige heterocyclische Stickstoffbase vom aromatischen Typ in quaternisierter Form.

Europäisches Patentamt

# EUROPÄISCHER RECHERCHENBERICHT

Nummer der Anmeldung

EP 91 11 7882

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | US-A-3 211 783 (O.F. HECHT)<br>--- | 1-3 | C07C309/42<br>C07C309/46<br>C07C309/14<br>C07C215/24<br>C25D3/12 |
| A | US-A-2 533 085 (F.F. BLICKE)<br>--- | 5 | |
| A | FR-A-1 215 544 (BADISCHE ANILIN & SODA FABRIK)<br>--- | 5 | |
| A | US-A-3 579 457 (R.M. LINCOLN ET AL)<br>----- | 5 | |
| | | | RECHERCHIERTE SACHGEBIETE (Int. Cl.5 ) |
| | | | C07C<br>C25D |

Der vorliegende Recherchenbericht wurde für alle Patentansprüche erstellt

| Recherchenort | Abschlußdatum der Recherche | Prüfer |
|---|---|---|
| DEN HAAG | 24 JANUAR 1992 | ZAROKOSTAS K. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)